# EUROPEAN PATENT APPLICATION

(11) **EP 4 389 733 A1**
(43) Date of publication of application: **26.06.2024**
(21) Application number: 21954084.6
(22) Date of filing: 24.12.2021
(51) Int. Cl.: C07C 51/09, C07C 63/70, C07C 227/18, C07C 229/56, C07C 43/225, C07C 41/18

(54) **METHOD FOR PREPARING 5-BROMO-2-CHLORO-BENZOIC ACID AS RAW MATERIAL IN HYPOGLYCEMIC DRUG SYNTHESIS**

(30) Priority: 16.08.2021 CN 202110937660
(71) Applicant: Zhejiang Ausun Pharmaceutical Co., Ltd., Taizhou, Zhejiang 317016 (CN)
(72) Inventor: LI, Yunfeng, Linhai, Zhejiang 317016 (CN); ZHENG, Zhiguo, Linhai, Zhejiang 317016 (CN)
(74) Representative: Bianchetti & Minoja with Trevisan & Cuonzo IPS SRL
(86) International application number: PCT/CN2021/141256
(87) International publication number: WO 2023/019849

(57) **Abstract**

The invention relates to a preparation method and application of 5-bromo-2-chloro-benzoic acid as a synthetic raw material of a hypoglycemic agent. Specifically, the 5-bromo-2-chloro-benzoic acid is prepared by taking a 5-bromo-2-aminobenzoic acid derivative as an initial raw material through two steps of reactions of diazotization, chlorination and hydrolysis, and the obtained product has the advantages of few isomer impurities, high reaction yield, good purity, low cost and suitability for industrial production. The invention also relates to the application of the 5-bromo-2-amino-benzoic acid in preparing antidiabetic drugs.

## Description

### Technical Field

The present invention relates to the field of synthesis of pharmaceutical intermediates, in particular to a preparation method and application of 5-bromo-2-chloro-benzoic acid as a synthetic raw material for hypoglycemic drugs.

### Background

The compound 5-bromo-2-chloro-benzoic acid of formula I is an important starting material for the preparation of the antidiabetic drugs Dapagliflozin and Empagliflozin.

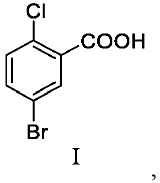

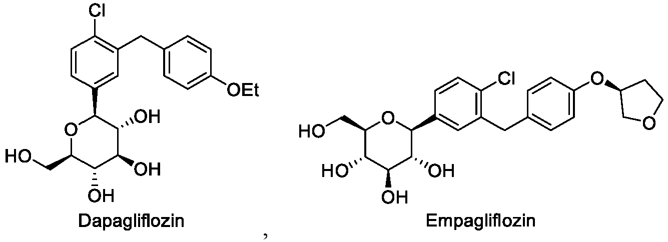

The reported preparation methods of the compound of formula I mainly include the following.

**Method I:** Chinese patent CN105622382B reports the preparation of the compound of formula I by bromination of 2-chloro-trichloromethyl-benzene followed by hydrolysis. However, the cost of the reaction raw material 2-chloro-trichloromethyl-benzene is relatively high, and it is difficult to be completely hydrolyzed. The reaction will produce a higher content of isomer impurity, which needs further recrystallization to remove it, thus affecting the yield and product purity.

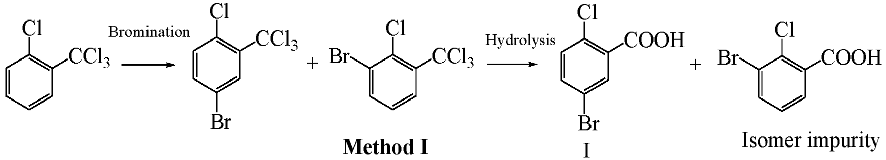

**Method II:** Chinese patents CN107954852A, CN110105193A and CN110590541A all report using 2-chlorobenzoic acid as raw material, and using different brominating agents such as sodium bromide and sodium periodate, bromine, N-bromosuccinimide, dibromohydantoin, etc. However, due to the nature of the reaction, about 10% of isomer impuriy will be produced during the bromination reaction, the yield and purity of the compound of formula I will be affected, and a lot of waste acid will be produced, making it unsuitable for industrial production.

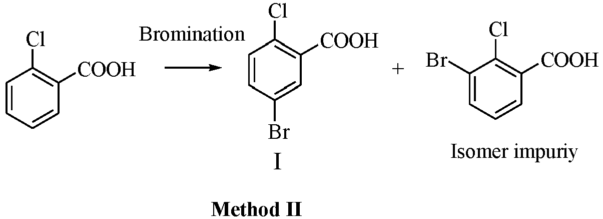

Chinese patent CN110002989B reports that the compound of formula I can be efficiently prepared by adding an inhibitor sodium sulfide, potassium sulfide or sodium sulfite to a reaction system using 2-chlorobenzoic acid as a raw material and NBS/sulfuric acid, and the generation of the impurity 4-bromo-2-chlorobenzoic acid is inhibited. However, it remains to be questioned whether inhibitors are still present in strong acid systems and whether the impurity 4-bromo-2-chlorobenzoic acid will be generated.

**Method III:** Chinese patent CN108250060A reports a method for preparing the compound of formula I from salicylic acid. Although this method can effectively reduce the isomer formation, the chlorination step uses a higher reaction temperature of 120-180 °C, which has higher requirements for equipment, and uses the environmentally-unfriendly chlorinated reagents such as carbon tetrachloride, boron trichloride, phosphorus trichloride, etc., so this method is not ideal.

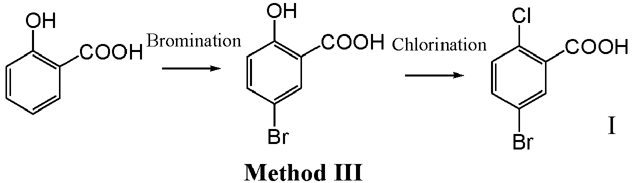

**Method IV:** Chinese patent CN111925289A reports the preparation of the compound of formula I by using 2-chlorobenzoic acid as raw material and sequentially carrying out chlorination, amidation, cyclization, bromination and hydrolysis. The reaction steps of the route are long, acid chloride reagent with strong corrosivity is used in the chlorination reaction, and dangerous butyl lithium reagent is used in the bromination reaction, so that the cost is higher, the production period is long, and the route is not suitable for industrial production.

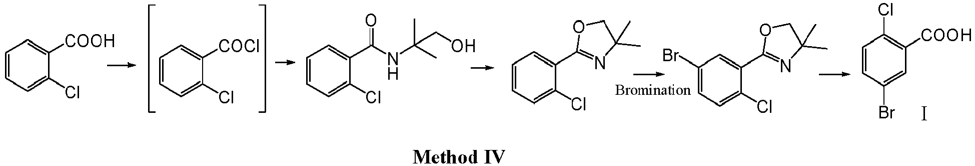

**Method V:** Chinese patent CN112979448A reports a method for preparing the compound of formula I by using 2-chlorobenzoic acid as a raw material and dibromoamino-silica gel as a brominating reagent. In said method, the cost of the amino silica gel raw material is high, and the method for preparing dibromoamino-silica gel uses bromine and more than 2 times the equivalent of potassium carbonate as acid binding agents. Although the amino silica gel can be recycled and reused, it is ultimately disposed of as solid waste. Moreover, the bromination reaction uses 1-chlorobutane and 1-bromobutane as reaction solvents, iron triflate as catalyst, and the reaction takes place at 60-70 °C, resulting in higher costs.

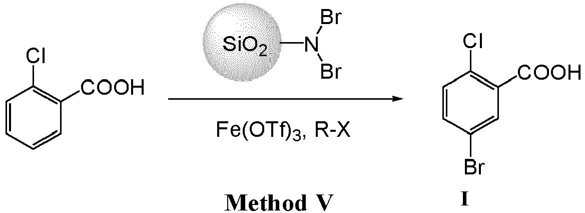

In summary, the currently reported preparation methods of the compound of formula I have problems of long reaction steps, not being environmentally friendly enough, high content of isomer impurities, high cost and the like, which bring certain difficulties to industrial scale production. Therefore, there is a need to further develop a simple and efficient synthetic method suitable for industrial production of the compound of formula I.

### Disclosure of Invention

In order to overcome the shortcomings and deficiencies of the prior art, the present invention provides a novel method for synthesizing the compound 5-bromo-2-chlorobenzoic acid of formula I. This method has the characteristics of convenient operation, cheap and easily available raw and auxiliary materials, high product yield, good purity of intermediates and target products, and is easy to carry out industrial production.

In a first aspect, the present invention provides a process for preparing a compound of formula I, which process comprises:
step (1): diazotizing and chlorinating the compound of formula III to prepare the compound of formula II, and
step (2): hydrolyzing the compound of formula II to obtain the compound of formula I, 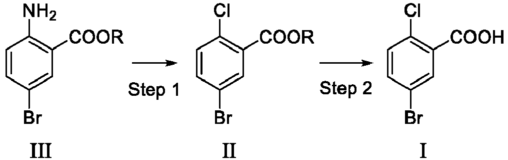 wherein:
   R is selected from: C₁₋₆alkyl, C₂₋₆alkenyl, C₂₋₆alkynyl, C₃₋₈cycloalkyl, C₃₋₈cycloalkyl-C₁₋₆alkyl, C₆₋₁₀aryl, C₆₋₁₀aryl-C₁₋₆alkyl, 5-10 membered heteroaryl, 3-12 membered heterocycloalkyl, 5-10 membered heteroaryl-C₁₋₆alkyl, 3-12 membered heterocycloalkyl-C₁₋₆alkyl, C₁₋₆alkoxycarbonyl, C₁₋₆alkoxycarbonyl-C₁₋₆alkyl, C₆₋₁₀aryl-C₁₋₆alkoxycarbonyl, C₆₋₁₀aryl-C₁₋₆alkoxycarbonyl-C₁₋₆alkyl, each of which is optionally substituted with one or more groups independently selected from halogen, amino, -NH(C₁₋₆alkyl), -N(C₁₋₆alkyl)₂, hydroxy, C₁₋₆alkyl, C₁₋₆alkoxy, halo-C₁₋₆alkyl, halo-C₁₋₆alkoxy, C₃₋₈cycloalkyl, C₆₋₁₀aryl, 5-10 membered heteroaryl, or 3-12 membered heterocycloalkyl;
   alternatively, when R is H, step (1) as described above is carried out to directly obtain the compound of formula I.

In a preferred embodiment, R is selected from: C₁₋₆alkyl, C₂₋₆alkenyl, C₃₋₈cycloalkyl, C₃₋₈cycloalkyl-C₁₋₆alkyl, C₆₋₁₀aryl, or C₆₋₁₀aryl-C₁₋₆alkyl, each of which is optionally substituted with one or more groups independently selected from halogen, amino, -NH(C₁₋₆alkyl), -N(C₁₋₆alkyl)₂, hydroxy, C₁₋₆alkyl, C₁₋₆alkoxy, halo-C₁₋₆alkyl, or halo-C₁₋₆alkoxy.

In another preferred embodiment, R is selected from: methyl, ethyl, propyl, isopropyl, allyl, cyclopropyl, n-butyl, isobutyl, tert-butyl, cyclobutyl, n-pentyl, n-hexyl, cyclohexyl, phenyl or benzyl.

In step (1), diazotizing and chlorinating the compound of formula III to prepare the compound of formula II refers to: the compound of formula III is first diazotized and then chlorinated to prepare the compound of formula II in two steps; or the compound of formula III is subjected to diazotization and chlorination reaction to prepare the compound of formula II in a one-pot method.

In the two-step reaction, the diazotization reaction refers to controlling the temperature below 20°C, for example -10 to 15°C, and adding a diazotization reagent to the compound of formula III in an acidic system to carry out the reaction.

The diazotization reagent may be selected from nitrous acid or its salts or C₁₋₆alkyl esters, such as nitrous acid, sodium nitrite, potassium nitrite, methyl nitrite, ethyl nitrite, isoamyl nitrite or tert-butyl nitrite.

The chlorination reaction is a reaction of chlorinating a diazotized group in a solution containing chloride ions in the presence of a copper catalyst. For example, the chlorination reaction may be carried out by chlorinating the diazotized group described above in a solution containing chloride ions at a temperature of -10 to 40 °C. The solution containing chloride ions can be selected from a solution of hydrochloric acid, sodium chloride, potassium chloride, cupric chloride, ferric chloride or ferrous chloride.

The copper catalyst is selected from metallic copper, cuprous chloride, cupric chloride, cuprous bromide or cuprous iodide.

In a particularly preferred embodiment, step (1) comprises: adding the compound of formula III into an acidic solvent, controlling the reaction temperature to be -10 to 15° C, and dropwise adding a diazotization reagent to obtain a diazotization reaction solution. And then controlling the temperature to be -10 to 40 °C, dropwise adding the diazotization reaction solution into a solution containing a copper catalyst and chloride ions, precipitating the compound of formula II, filtering and washing to obtain the compound of formula II. The product is directly subjected to the next reaction without drying.

In another preferred embodiment, in step (1), the compound of formula III is prepared into a compound of formula II by a one-pot process comprising: adding the compound of formula III into an acidic reaction solution containing a copper catalyst and chloride ions, stirring until uniform, controlling the reaction temperature to be -10 to 70 °C, and dropwise adding a diazotization reagent for reaction to obtain the compound of formula II. The product is filtered and washed with water, and then directly subjected to the next reaction.

In the case that the copper catalyst is a metallic copper, after the product is filtered, the compound of formula II is dissolved in a solvent selected from toluene, hexane, n-heptane, methanol, ethanol or isopropanol or a mixed solution thereof with water, and then filtered to obtain the metallic copper catalyst, which is recycled and used. And then the solution of the compound of formula II is distilled to dryness and subjected to the next reaction directly, or the compound of formula II is crystallized, centrifuged and subjected to the next reaction directly.

In step (2), the compound of formula II is hydrolyzed in an aqueous alkali metal hydroxide solution to obtain the compound of formula I.

In a preferred embodiment, the compound of formula II is hydrolyzed in an aqueous alkali metal hydroxide solution, the water insoluble impurities are removed by extraction with an organic solvent, followed by acidification, centrifugation, and drying to obtain the compound of formula I.

The aqueous alkali metal hydroxide solution may be selected from aqueous lithium hydroxide, aqueous sodium hydroxide or aqueous potassium hydroxide, or mixtures thereof. The organic solvent used for removing impurities may be selected from non-polar or polar aprotic organic solvents, such as hexane, n-heptane, isopropyl acetate, toluene, xylene or chlorobenzene, or mixtures of two or more thereof.

The acid used for said acidification is selected from hydrochloric acid, hydrobromic acid, hydroiodic acid, sulfuric acid, phosphoric acid, nitric acid, formic acid or acetic acid, or a mixture of two or more thereof.

In a second aspect, the present invention provides a process for preparing a compound of formula III, 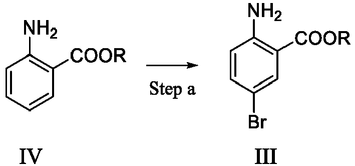 comprising:
step (a): reacting the compound of formula IV with a brominating reagent to obtain the compound of formula III, wherein R is as defined above for the first aspect.

In a preferred embodiment, the process comprises: the compound of formula IV is dissolved in a polar solvent, the reaction temperature is controlled to be, for example, 5 to 50 °C, and a brominating reagent or a solution thereof is added dropwise to carry out a reaction to obtain the compound of formula III. After the reaction is finished, adding the reaction solution into tap water to precipitate the compound of formula III, which can be used directly to prepare the compound of formula II after centrifugation.

The polar solvent is selected from: acetonitrile, tetrahydrofuran, methyltetrahydrofuran, N,N-dimethylformamide, N,N-diethylformamide, N-methylpyrrolidone, dimethyl sulfoxide, formic acid, acetic acid or water, or a mixture of two or more thereof.

The brominating reagent is selected from: bromine, hydrobromic acid, lithium bromide, sodium bromide, potassium bromide, sodium bromate, dibromohydantoin, N-bromoacetamide, N-bromosuccinimide, phenyltrimethylammonium tribromide, (bromomethyl) triphenylphosphonium bromide, or a mixture of two or more thereof.

In a third aspect, the present invention provides a process for preparing a compound of formula I, which process comprises steps (a), (1) and (2) as described above: 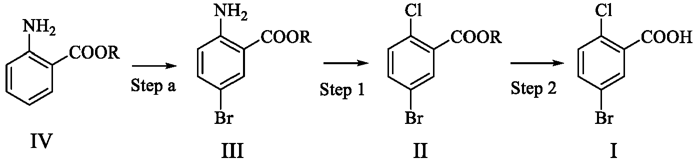 wherein R is as defined above.

The reaction conditions and preferred embodiments of steps (a), (1) and (2) are as defined above.

In a fourth aspect, the present invention provides a process for preparing a compound of formula VI, comprising the steps of: 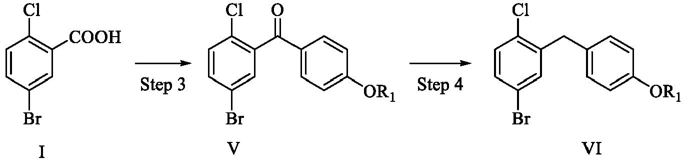 step (3): reacting the compound of formula I with the compound of formula VII through Friedel-crafts acylation reaction to prepare the compound of formula V, 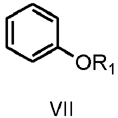
wherein R₁ is selected from: H, C₁₋₆alkyl, such as methyl or ethyl, 3-tetrahydrofuranyl, 3-S-tetrahydrofuranyl or 3-R-tetrahydrofuranyl; and
step (4): reducing the compound of formula V to obtain the compound of formula VI.

In a preferred embodiment, the present invention provides a process for preparing a compound of formula VI, comprising steps (1), (2), (3) and (4): 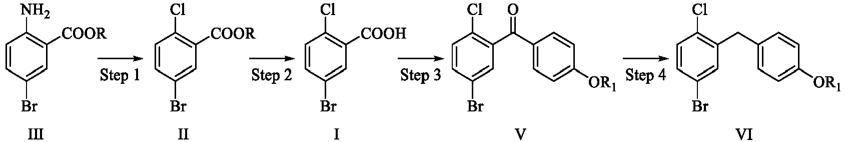
wherein R₁ is selected from: H, C₁₋₆alkyl, for example methyl or ethyl, 3-tetrahydrofuranyl, 3-S-tetrahydrofuranyl or 3-R-tetrahydrofuranyl, and R is as defined for the first aspect,
wherein steps (1), (2), (3) and (4) are as defined above.

In a preferred embodiment, the process comprises: (1) diazotizing and chlorinating the compound of formula III to prepare the compound of formula II, (2) hydrolyzing the compound of formula II to obtain the compound of formula I, (3) reacting the compound of formula I with the compound of formula VII through Friedel-crafts acylation reaction to prepare the compound of formula V, and (4) reducing the compound of formula V to obtain the compound of formula VI.

The reaction conditions and preferred embodiments of steps (1) and (2) are as defined above.

In the process, preferably, step (3) comprises: the compound of formula I is converted into an acyl chloride under the action of an acylating reagent in a weakly polar solvent, and then reacted with the compound of formula VII in a weakly polar solvent under the action of a Lewis acid to obtain the compound of formula V.

In a more preferred embodiment, step (3) comprises: the compound of formula I is converted into an acyl chloride under the action of an acylating reagent in a weakly polar solvent, and then reacted with the compound of formula VII in a weakly polar solvent under the action of a Lewis acid, the reaction temperature is controlled to be -10 to 20 °C until the reaction is finished, and the reaction is concentrated and crystallized to obtain the compound of formula V. Specific work-up of the reaction may include: adding water to quench, washing the organic phase with water, concentrating, crystallizing, centrifuging, and drying to obtain the compound of formula V.

The acylating reagent is selected from oxalyl chloride, thionyl chloride, phosphorus trichloride, phosphorus pentachloride or phosphorus oxychloride.

The Lewis acid is selected from aluminum chloride, ferric chloride, cupric chloride or aluminum bromide.

The weakly polar solvent is selected from tetrahydrofuran, methyltetrahydrofuran, dichloromethane, chloroform, isopropyl ether or methyl tert-butyl ether, or a mixture of two or more thereof.

The solvent used for crystallization is selected from toluene, hexane, n-heptane, methanol, ethanol, isopropanol, dichloromethane, isopropyl ether, methyl tert-butyl ether or water, or a mixture of two or more thereof.

In the process, step (4) comprises: dissolving the compound of formula V in an organic solvent, and reducing under the action of a reducing agent and an auxiliary agent to obtain the compound of formula VI.

In a more preferred embodiment, step (4) comprises: dissolving the compound of formula V in an organic solvent, controlling the temperature to be -10 to 80 °C and reducing under the action of a reducing agent and an auxiliary agent, and crystallizing to obtain the compound of formula VI.

The organic solvent is selected from methanol, ethanol, isopropanol, acetonitrile, isopropyl ether, methyl tert-butyl ether, tetrahydrofuran, methyl tetrahydrofuran, toluene, chlorobenzene, pentane, hexane, cyclohexane, n-heptane or water, or a mixture of two or more of the above.

The reducing agent is selected from lithium borohydride, sodium borohydride, potassium borohydride, borane, triethylsilane, trimethylsilane, tetramethyldisiloxane or lithium aluminum hydride.

The auxiliary agent is selected from aluminum chloride, ferric chloride, cupric chloride, aluminum bromide, trifluoroacetic acid, trifluoromethanesulfonic acid or boron trifluoride diethyl etherate.

The solvent used for crystallization is selected from methanol, ethanol, isopropanol, acetonitrile, isopropyl ether, methyl tert-butyl ether, tetrahydrofuran, methyl tetrahydrofuran, toluene, chlorobenzene, pentane, hexane, cyclohexane, n-heptane or water, or a mixture of two or more thereof.

In another preferred embodiment, the present invention provides a process for preparing a compound of formula VI, comprising steps (a), (1), (2), (3) and (4): 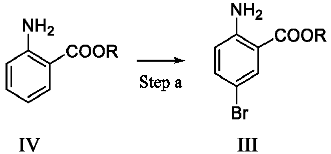 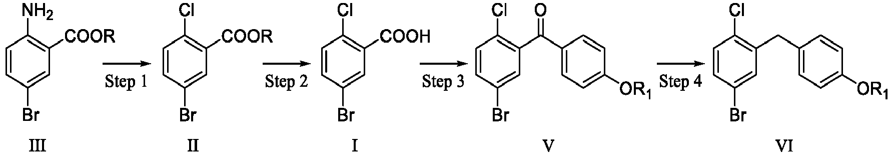 wherein steps (a), (1), (2), (3) and (4) and preferred embodiments are as defined above, and R and R₁ are as defined above.

In a fifth aspect, the invention provides the use of a compound of formula III in the preparation of the hypoglycemic drugs dapagliflozin or engagliflozin.

In particular, the present invention provides a process for preparing dapagliflozin or engagliflozin, the process comprises any one or more of the steps as described in the first, second, third and fourth aspects above, i.e. the process comprises any one or more of the steps (a), (1), (2), (3) and (4) described above, wherein R and R₁ are as defined above.

For example, the present invention provides a process for preparing dapagliflozin, which comprises the step of preparing a compound of formula VI from a compound of formula III, 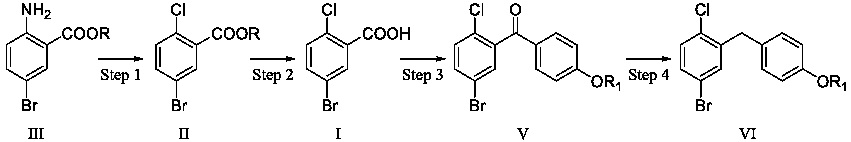 wherein steps (1), (2), (3) and (4) are as defined above, and R and R₁ are as defined above.

Starting from the compound of formula VI, reference is made to the processes of patent applications WO03099836A1 and WO2005092877A1 for the preparation of dapagliflozin and engagliflozin, respectively. Patent applications WO03099836A1 and WO2005092877A1 are incorporated herein by reference in their entirety.

In a specific embodiment, the present invention provides a process for preparing dapagliflozin, comprising the steps of: 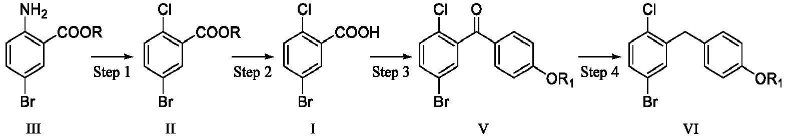 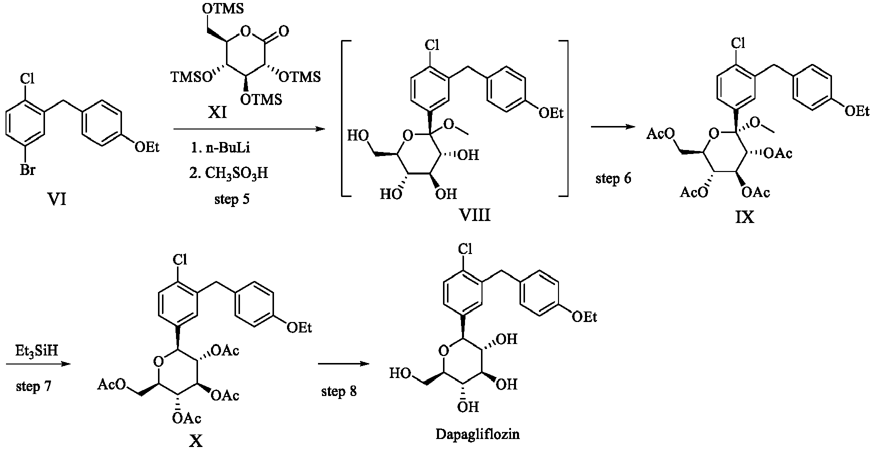 wherein steps (1), (2), (3) and (4) are as defined above, R is as defined above, R₁ is ethyl, and steps (5), (6), (7) and (8) are as described in the examples of WO03099836A1.

Compared with the prior art, the present invention has the following advantages:
1. The compound of formula I prepared by the present invention has high yield, good purity and less isomer impurities;
2. The process for preparing the compound of formula I according the present invention has few steps and simple process, and the used solvent, catalyst and acid can be recycled and reused, so that the cost is obviously reduced and the process is suitable for industrial production;
3. The key intermediate compound of formula VI of the hypoglycemic drugs prepared by using the compound of formula I according to the present invention has high yield and a purity of 99.9%, thereby providing reliable quality assurance for the preparation of the final drug.

### Definition

For the purpose of interpreting this specification, the following definitions will be used, and where appropriate, terms used in the singular may also include the plural, and vice versa. It is to be understood that the terminology used herein is for the purpose of describing particular embodiments only, and is not intended to be limiting.

The term "halogen" or "halo" as used herein means F, Cl, Br or I. Furthermore, the term "halogen-substituted" group is intended to include mono- or polyhalogenated groups in which one or more hydrogen atoms in the groups are replaced with one or more same or different halogens.

The term "alkyl" as used herein refers to a straight or branched chain saturated hydrocarbon group consisting of carbon atoms and hydrogen atoms. In particular, the alkyl group has 1-10, such as 1 to 6, 1 to 5, 1 to 4, 1 to 3, or 1 to 2 carbon atoms. For example, as used herein, the term "C₁-C₆alkyl" refers to a straight or branched chain saturated hydrocarbon group having 1 to 6 carbon atoms, examples of which are methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylpentyl and the like.

The term "alkenyl" as used herein refers to a straight or branched chain unsaturated hydrocarbon group consisting of carbon atoms and hydrogen atoms and containing at least one double bond. In particular, the alkenyl group has 2-8, such as 2 to 6, 2 to 5, 2 to 4, or 2 to 3 carbon atoms. For example, as used herein, the term "C₂-C₆alkenyl" refers to a straight or branched chain alkenyl group having 2 to 6 carbon atoms, such as ethenyl, propenyl, allyl, butenyl, pentenyl, and the like.

The term "alkynyl" as used herein refers to a straight or branched chain unsaturated hydrocarbon group consisting of carbon atoms and hydrogen atoms and containing at least one triple bond. In particular, the alkynyl group has 2-8, e.g., 2 to 6, 2 to 5, 2 to 4, or 2 to 3 carbon atoms. For example, as used herein, the term "C₂-C₆alkynyl" refers to a straight or branched chain alkynyl group having 2 to 6 carbon atoms, such as ethynyl, propynyl, propargyl, butynyl, and the like.

The term "alkoxy" as used herein means the group -O-alkyl, wherein alkyl has the meaning described herein. In particular, such term includes the group -O-C₁₋₆alkyl, more specifically -O-C₁₋₃alkyl. Representative examples of alkoxy group include, but are not limited to, methoxy, ethoxy, propoxy (including n-propoxy, isopropoxy), butoxy (including n-butoxy, isobutoxy, tert-butoxy), pentoxy (including n-pentoxy, isopentoxy, neopentoxy), hexoxy (including n-hexoxy, isohexoxy), and the like.

The term "halo-C₁-C₆alkyl" or "halogen-substituted C₁-C₆alkyl" as used herein refers to C₁-C₆alkyl groups described above in which one or more (e.g., 1, 2, 3, 4, or 5) hydrogen atoms are replaced with a halogen. It will be understood by those skilled in the art that when there is more than one halogen substituent, the halogens may be the same or different and may be located on the same or different C atoms. Examples of "halo-C₁-C₆alkyl" or "halogen-substituted C₁-C₆alkyl" include, for example, -CH₂F, -CHF₂, -CF₃, -CCl₃, -C₂F₅, -C₂Cl₅, -CH₂CF₃, -CH₂Cl, -CH₂CH₂CF₃ or -CF(CF₃)₂, and the like.

The term "cycloalkyl" as used herein refers to a monocyclic, fused polycyclic, bridged polycyclic, or spiro non-aromatic saturated monovalent hydrocarbon ring structure having the indicated number of ring atoms. The cycloalkyl group can have 3 to 12 carbon atoms (i.e., C₃-C₁₂cycloalkyl), for example, 3 to 10, 3 to 8, 3 to 7, 3 to 6, 5 to 6 carbon atoms. Examples of suitable cycloalkyl groups include, but are not limited to, monocyclic structures such as cyclopropyl, cyclobutyl, cyclopentyl, cyclohexyl, cycloheptyl, or cyclooctyl; or polycyclic (e.g., bicyclic) structures, including spiro, fused, or bridged systems, such as bicyclo[1.1.1]pentyl, bicyclo[2.2.1]heptyl, spiro[3.4]octyl, bicyclo[3.1.1]hexyl, bicyclo[3.1.1]heptyl, or bicyclo[3.2.1]octyl, and the like.

The term "cycloalkyl" as used herein also includes "cycloalkenyl". "Cycloalkenyl" means a monocyclic, fused polycyclic, bridged polycyclic, or spiro non-aromatic unsaturated hydrocarbon ring structure having the indicated number of ring atoms and containing at least one (e.g., 1, 2, or 3) carbon-carbon double bond. Cycloalkenyl groups can have 3 to 12 carbon atoms (i.e., C₃-C₁₂cycloalkenyl), for example, 3 to 10, 3 to 8, 3 to 7, 3 to 6, 5 to 6 carbon atoms. Examples of suitable cycloalkenyl groups include, but are not limited to, monocyclic structures such as cyclopropenyl, cyclobutenyl, cyclopentenyl, cyclopentadienyl, cyclohexenyl, cyclohexadienyl, cycloheptenyl, cycloheptadienyl, cycloheptatrienyl, or cyclooctenyl.

The term "heterocycloalkyl" as used herein refers to a monocyclic, fused polycyclic, spiro or bridged polycyclic non-aromatic saturated ring structure having one or more (e.g., 1, 2, 3, or 4) heteroatoms independently selected from O, N and S and the indicated number of ring atoms, or N-oxides thereof, or S-oxides or S-dioxides thereof. The heterocycloalkyl group can have 3 to 12 ring members (can be referred to as 3-12 membered heterocycloalkyl), for example 3 to 10 ring members, 3 to 8 ring members, 3 to 7 ring members, 4 to 6 ring members, 5 to 6 ring members. Heterocycloalkyl groups typically contain up to 4 (e.g., 1, 2, 3, or 4) heteroatoms. Examples of suitable heterocycloalkyl groups include, but are not limited to, azetidinyl, oxetanyl, thietanyl, pyrrolidinyl (e.g., 1-pyrrolidinyl, 2-pyrrolidinyl, and 3-pyrrolidinyl), tetrahydrofuryl (e.g., 1-tetrahydrofuryl, 2-tetrahydrofuryl, and 3-tetrahydrofuryl), tetrahydrothienyl (e.g., 1-tetrahydrothienyl, 2-tetrahydrothienyl, and 3-tetrahydrothienyl), piperidinyl (e.g., 1-piperidinyl, 2-piperidinyl, 3-piperidinyl, and 4-piperidinyl), tetrahydropyranyl (e.g., 4-tetrahydropyranyl), tetrahydrothiopyranyl (e.g., 4-tetrahydrothiopyranyl), morpholinyl (e.g., morpholino), thiomorpholinyl, dioxanyl, piperazinyl, or azepanyl, diazepanyl, such as, 1,4-diazepanyl, 3,6-diaza-bicyclo[3.1.1]heptyl or 3-aza-bicyclo[3.2.1]octyl. The atom of the heterocycloalkyl group that is connected to the rest of the compound can be a carbon atom or a heteroatom, as long as it is chemically feasible.

The term "heterocycloalkyl" as used herein also includes "heterocycloalkenyl" and refers to a "heterocycloalkyl" as defined herein containing at least one (e.g., 1, 2, or 3) double bond, such as pyrrolinyl (e.g., 1-pyrrolinyl, 2-pyrrolinyl, 3-pyrrolinyl, 4-pyrrolinyl, or 5-pyrrolinyl), dihydrofuranyl (e.g., 1-dihydrofuranyl, 2-dihydrofuranyl, 3-dihydrofuranyl, 4-dihydrofuranyl, or 5-dihydrofuranyl), dihydrothienyl (e.g., 1-dihydrothienyl, 2-dihydrothienyl, 3-dihydrothienyl, or 4-dihydrothienyl), tetrahydropyridinyl (e.g., 1-, 2-, 3-, 4-, 5-, or 6-tetrahydropyridinyl), tetrahydropyranyl (e.g., 4-tetrahydropyranyl) or tetrahydrothiopyranyl (e.g., 4-tetrahydrothiopyranyl).

The term "aryl" as used herein means a monovalent aromatic hydrocarbon radical derived by removal of one hydrogen atom from a single carbon atom in an aromatic ring system. In particular, aryl refers to a monocyclic or fused polycyclic aromatic ring structure having the indicated number of ring atoms. In particular, such term includes groups comprising 6 to 14, such as 6 to 10, preferably 6 ring atoms. Specific aryl groups include phenyl and naphthyl, with the most specific aryl group being phenyl.

The term "heteroaryl" as used herein means a monocyclic or fused polycyclic aromatic ring structure having one or more (e.g., 1, 2, 3 or 4) heteroatoms independently selected from O, N and S and the indicated number of ring atoms, or N-oxides thereof, or S-oxides or S-dioxides thereof. In particular, the aromatic ring structure may have 5 to 10 ring members. Heteroaryl groups can be, for example, a 5-6 membered monocyclic ring, or a fused bicyclic ring structure formed by two fused 6-membered rings, two fused 5-membered rings, fused 6- and 5-membered ring, or fused 5- and 4-membered ring. Heteroaryl rings will typically contain up to 4 heteroatoms, more typically up to 3 heteroatoms, more typically up to 2, for example a single heteroatom independently selected from O, N and S, wherein N and S may be in oxidation states such as N-oxide, S=O or S(O)₂. In one embodiment, the heteroaryl ring contains at least one ring nitrogen atom, at least one ring sulfur atom, or at least one ring oxygen atom. For example, the heteroaryl group can be a fused ring containing 1, 2, 3, or 4 heteroatoms independently selected from N, O or S, such as benzofuran, benzothiophene, indole, benzimidazole, indazole, benzotriazole, pyrrolo[2,3-b]pyridine, pyrrolo[2,3-c]pyridine, pyrrolo[3,2-c]pyridine, pyrrolo[3,2-b]pyridine, imidazo[4,5-b]pyridine, imidazo[4,5-c]pyridine, pyrazolo[4,3-d]pyridine, pyrazolo[4,3-c]pyridine, pyrazolo[3,4-c]pyridine, pyrazolo[3,4-b]pyridine, isoindole, purine, indolizine, imidazo[1,2-a]pyridine, imidazo[1, 5-a]pyridine, pyrazolo[1,5-a]pyridazine, pyrrolo[1,2-b]pyrimidine, imidazo[1,2-c]pyrimidine, 5H-pyrrolo[3,2-b]pyrazine, 1H-pyrazolo[4,3-b]pyrazine, 1H-pyrazolo[3,4-d]pyrimidine, 7H-pyrrolo[2,3-d]pyrimidine, quinoline, isoquinoline, cinnoline, quinazoline, quinoxaline, phthalazine, 1,6-naphthyridine, 1,7-naphthyridine, 1,8-naphthyridine, 1,5-naphthyridine, 2,6-naphthyridine, 2,7-naphthyridine, pyrido[3,2-d]pyrimidine, pyrido[4,3-d]pyrimidine, pyrido[3,4-d]pyrimidine, pyrido[2,3-d]pyrimidine, pyrido[2,3-b]pyrazine, pyrido[3,4-b]pyrazine, pyrimido[5,4-d]pyrimidine, pyrazino[2,3-b]pyrazine and pyrimido[4,5-d]pyrimidine. For example, the heteroaryl group can be a 5-6 membered heteroaryl group containing 1 or 2 heteroatoms independently selected from N, O or S. Examples of suitable 5-membered monocyclic heteroaryl groups include, but are not limited to, pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl, and tetrazolyl; examples of suitable 6-membered monocyclic heteroaryls include, but are not limited to, pyridinyl, pyrazinyl, pyridazinyl, pyrimidinyl and triazinyl. The atom of the heteroaryl group that is connected to the rest of the compound can be a carbon atom or a heteroatom, as long as it is chemically feasible.

When a substituent is described as "optionally substituted", it is meant that the group may be unsubstituted or substituted with one or more (e.g., 0, 1, 2, 3, 4, or 5 or more, or any range derivable therein) substituents listed for that group, which substituents may be the same or different. In one embodiment, an optionally substituted group is substituted with 1 substituent. In another embodiment, the optionally substituted group is substituted with 2 substituents. In another embodiment, the optionally substituted group is substituted with 3 substituents. In another embodiment, the optionally substituted group is substituted with 4 substituents.

As used herein, the terms "comprises" or "comprising" are intended to be inclusive of the stated elements, integers, or steps, but not to exclude any other elements, integers, or steps. When the term "comprising" or "comprises" is used herein, unless otherwise indicated, the combination of the recited elements, integers or steps is also encompassed.

### Detailed Description

The process of the present invention is further illustrated by the following examples. It should be understood that the following examples are provided only for the purpose of enabling a better understanding of the present invention, and are not intended to limit the scope of the present invention in any way.

Unless otherwise stated, the following solvents and reagents are commercially available. The starting materials used are either commercially available or prepared by further processing via conventional reactions well known in the art.

### Preparation of the compound of formula III

### Example 1: Preparation of ethyl 5-bromo-2-amino-benzoate

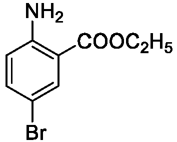

To 175kg of tetrahydrofuran was added 41.5kg of ethyl 2-aminobenzoate, stirred to form a clear solution, and 44.5kg of N-bromosuccinimide was added portionwise while keeping the temperature of the reaction pot at 10 - 35°C. After the addition was complete, the reaction was stirred for 30-60 minutes. Saturated aqueous sodium bisulfite solution (2.5kg) was added, and then tetrahydrofuran was recovered by distillation under reduced pressure, which can be reused. 150kg of tap water was added into the concentration pot to form slurry, which was centrifuged and washed with 30kg of tap water. 73.2 kg of wet product of the compound ethyl 5-bromo-2-aminobenzoate of formula III was obtained, which was directly used in the next reaction without drying. The yield was calculated as 100% and the purity was 99.1%.

### Example 2: Benzyl 5-bromo-2-amino-benzoate

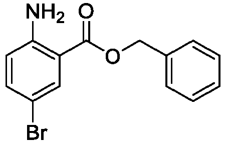

To 900g of DMF was added 227g of allyl 2-aminobenzoate, stirred to form a clear solution, and 163g of bromine was added portionwise while keeping the temperature of the reaction pot at 10 - 35°C. After the addition was complete, the reaction was stirred for 40-60 minutes. The reaction solution was added into an aqueous solution containing 35kg of sodium sulfite in 350g of tap water, the precipitated solid was separated by centrifugation and washed with 50g of tap water. 346g of wet product of benzyl 5-bromo-2-aminobenzoate was obtained, which was directly used in the next reaction without drying. The yield was calculated as 100% and the purity was 99.1%.

### Example 3: Preparation of allyl 5-bromo-2-amino-benzoate

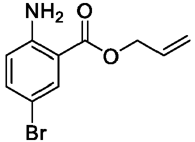

To 1000g of DMF was added 176.2g of allyl 2-aminobenzoate, stirred to form a clear solution, and 205g of dibromohydantoin was added portionwise while keeping the temperature of the reaction pot at 10 - 35°C. After the addition was complete, the reaction was stirred for 40-60 minutes. The reaction solution was added into an aqueous solution containing 30 g of sodium sulfite in 300g of tap water, the precipitated solid was separated by centrifugation and washed with 20g of tap water. 289.3g of wet product of allyl 5-bromo-2-aminobenzoate was obtained, which was directly used in the next reaction without drying. The yield was calculated as 100% and the purity was 99.2%.

### Preparation of the compound of formula I

### Example 4: Preparation of 5-bromo-2-chloro-benzoic acid

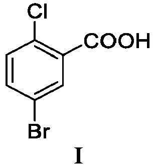

### Method 1:

To 300kg of 20% hydrochloric acid solution was added 73.2kg of the wet ethyl 5-bromo-2-aminobenzoate prepared in Example 1, then 8.0kg of copper powder was added, and after stirring evenly, 40kg of sodium nitrite aqueous solution (containing 17.6kg of sodium nitrite) was added dropwise while keeping the reaction temperature at 0 - 20 °C. After the addition was complete, stirring was continued for 30 minutes. Then 80kg of toluene was added to the reaction pot, stirred and filtered, and the copper catalyst was recovered, which can be reused. Layers were separated, and the acidic aqueous phase was reserved for reuse in the next batch. The organic phase was washed once with 20kg of tap water, concentrated to dryness to recover toluene, which can be reused. To the residue was added 80kg hexane, heated to 50 - 55°C, stirred to form a clear solution, the solution was cooled to form crystals, stirred at 0 - 10 °C for 30 minutes, and centrifuged to obtain 72.5kg of ethyl 5-bromo-2-chlorobenzoate wet product with a purity of 99.6%.

To a reaction pot was added 72.5kg of the compound of formula II obtained above, then 150kg of tap water was added, and 38.0kg of 30% NaOH solution was added dropwise while keeping the temperature at 40 - 55 °C, stirred to form a solution, and 31.0kg of concentrated hydrochloric acid was added dropwise to precipitate a solid, centrifuged, and dried at 50 - 70 °C for 6-8 hours to obtain 53.7kg of the compound of formula I. The total yield over three steps was 91.2%, and the purity was 99.7%.

### Method 2:

To 120kg of 20% hydrochloric acid solution was added 73.2kg of the wet ethyl 5-bromo-2-aminobenzoate prepared in Example 1, and after stirring evenly, 40kg of sodium nitrite aqueous solution (containing 17.6kg of sodium nitrite) was added dropwise while keeping the reaction temperature at -5 to 6 °C. After the addition was complete, stirring was continued for 30 minutes to obtain a solution of diazo compound of ethyl 5-bromo-2-aminobenzoate.

To a separate reaction pot was added 100kg of concentrated hydrochloric acid and 12.4kg of cuprous chloride, stirred to dissolve and the solution of diazo compound obtained above was added dropwise while keeping the temperature at 20 - 30 °C, and a large amount of solid was precipitated. The solid was filtered, and the filtrate was treated as waste liquid after being reused twice. The filter cake was washed with water and centrifuged to obtain 71.8kg of a wet product of ethyl 5-bromo-2-chlorobenzoate with a purity of 99.5%.

To a reaction pot was added 71.8kg of ethyl 5-bromo-2-chlorobenzoate obtained above, then 160kg of tap water was added, and 38.0kg of 30% NaOH solution was added dropwise while keeping the temperature at 40 - 55 °C, stirred to form a solution, and 31.0kg of concentrated hydrochloric acid was added dropwise to precipitate a solid, centrifuged, and dried at 50 - 70 °C for 8 hours to obtain 52.9kg of 5-bromo-2-chloro-benzoic acid, the compound of formula I. The total yield over three steps was 89.8%, and the purity was 99.6%.

### Method 3:

To 1050g of 20% hydrochloric acid solution was added 346 g of the wet benzyl 5-bromo-2-aminobenzoate prepared in Example 2, and after stirring evenly, 212 g of sodium nitrite aqueous solution (containing 70.4 g of sodium nitrite) was added dropwise while keeping the reaction temperature at -5 to 10 °C. After the addition was complete, stirring was continued for 30 minutes to obtain a solution of diazo compound of ethyl 5-bromo-2-aminobenzoate.

To a separate reaction flask was added 150g of concentrated hydrochloric acid and 50 g of cuprous chloride, stirred to dissolve and 260g of toluene was added. The solution of diazo compound obtained above was added dropwise while keeping the temperature at 20 - 40 °C. After the addition was complete, stirring was continued for 60 minutes. Layers were separated, the aqueous phase was retained for processing, and the organic phase was washed with 100g of ×2 tap water. Then 1200g of tap water was added, heated to 45 - 55° C, 190g of 30% NaOH solution was added dropwise and stirred to dissolve. 200g of concentrated hydrochloric acid was added dropwise to precipitate out a solid, centrifuged and dried at 50 - 70 °C for 6-9 hrs to obtain 198g of 5-bromo-2-chloro-benzoic acid, the compound of formula I. The total yield over three steps was 87.2%, and the purity was 99.4%.

According to Method 3, 207.7g of 5-bromo-2-chlorobenzoic acid of formula I was prepared in a yield of 88.2% and a purity of 99.7% using the wet allyl 5-bromo-2-aminobenzoate prepared in Example 3 as a starting material.

### Preparation of the compounds of formula VI

### Example 5: Preparation of 5-bromo-2-chloro-4' -ethoxydiphenylmethane

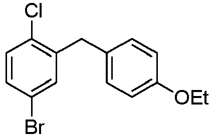

To 600kg of dichloromethane were added 235.5kg of 5-bromo-2-chlorobenzoic acid and 2kg of DMF successively, and then 130kg of oxalyl chloride was added dropwise while keeping the reaction temperature at 5 - 30 °C. After the addition was complete, the reaction was stirred to form a clear solution. Dichloromethane was recovered by distillation under normal pressure and used in the next batch for this step. After distillation, 200kg of dichloromethane was added to obtain an acyl chloride solution, which was stirred to form a clear solution.

To a reaction pot was added 1000kg of dichloromethane, 133kg of anhydrous aluminum trichloride and 123kg of phenetole, and the dichloromethane solution of acyl chloride prepared above was added dropwise while keeping the reaction temperature at -10 to 10 °C. After the addition was complete, the reaction was stirred for 1 hour. 10kg of drinking water was added to quench the reaction, and aluminum trichloride hydrate was precipitated, filtered and recovered. The filtrate was washed with drinking water twice (150kg×2), and dichloromethane was recovered under normal pressure, which can be reused after dehydration. Then 600kg of 95% ethanol was added, heated to form a clear solution, then the solution was cooled to precipitate crystals, centrifuged at 5 - 10 °C to obtain a wet product, which was dried at 45 - 55 °C to obtain 279.2kg of 5-bromo-2-chloro-4'-ethoxybenzophenone, the compound of formula V, with a yield of 82.4% and a purity of 99.8%.

To 600kg of methanol was added 279.2kg of the compound of formula V, 5-bromo-2-chloro-4'-ethoxybenzophenone, then 30kg of sodium borohydride was added portionwise. After the addition was complete, the mixture was stirred at 10 - 30 °C for 1 hour, then 10kg of aluminum trichloride was added, and the mixture was heated to 55 - 64 °C and reacted for 6-8 hours. After the reaction was complete, methanol was recovered under reduced pressure and can be reused in this step. After the distillation was finished, the residue was taken up in 450kg of ethyl acetate and washed with 120kg×2 of tap water. Layers were separated, the organic phase was concentrated to dryness, and the solvent can be reused in this step. The residue was taken up in 375kg of methanol and heated to 45 - 55°C to form a clear solution. The solution was cooled to precipitate crystals, centrifuged, and dried to dryness at 27 - 32 °C under reduced pressure to obtain 249.0kg of 5-bromo-2-chloro-4'-ethoxydiphenylmethane, the compound of formula VI, with a yield of 93.0% and a purity of 99.9%.

### Example 6: Preparation of (3S)-3-[4-[(5-bromo-2-chlorophenyl)methyl]phenoxy]tetrahydrofuran

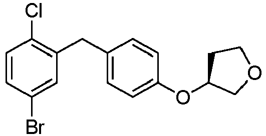

To 300kg of dichloromethane was added 141.3kg of 5-bromo-2-chlorobenzoic acid, 78kg of thionyl chloride was added dropwise while keeping the reaction temperature at -5 to 25° C. After the addition was complete, the mixture was stirred to obtain a clear solution. Dichloromethane was recovered by distillation under reduced pressure and can be reused in this step in the next batch. After the distillation was finished, 150kg of dichloromethane was added to obtain an acyl chloride solution, which was stirred to form a clear solution.

To a reaction pot were added 600kg of dichloromethane, 80kg of anhydrous aluminum trichloride and 98.0kg of (3S)-phenoxyl-tetrahydrofuran, and the dichloromethane solution of acyl chlorid prepared above was added dropwise while keeping the reaction temperature at -10 to 10 °C. After the addition was complete, the mixture was stirred for 1 to 2 hours. 6.0kg of drinking water was added to quench the reaction, and the precipitated aluminum trichloride hydrate was filtered and recovered. The filtrate was washed with drinking water (900kg × 2), dichloromethane was recovered by distillation under reduced pressure and can be reused after dehydration. To the concentration pot was added 360kg of methanol, heated to form a clear solution, and then the solution was cooled to precipitate crystals, centrifuged at 0 - 10 °C to obtain a wet product, which was dried at 40 - 50 °C to obtain 185.2kg of the compound of formula V, (R)-3-(4-(2-chloro-5-iodobenzoyl)phenoxy)tetrahydrofuran, with a yield of 81.0% and a purity of 99.7% of.

To 420kg of methanol was added 185.2kg of the compound V, (R)-3-(4-(2-chloro-5-iodobenzoyl)phenoxy)tetrahydrofuran, and then 18.0kg of sodium borohydride was added portionwise. After the addition was complete, the mixture was stirred at 10 - 30 °C for 1.5 hours, and then 15kg of boron trifluoride diethyl etherate was added, and the mixture was heated to 50 - 64°C and reacted for 5-7 hours. After the reaction was finished, methanol was recovered under reduced pressure and can be reused in this step. After the distillation was finished, 300kg of toluene was added and washed with tap water (70kg × 2). Layers were separated, the organic phase was concentrated to dryness, and the solvent can be reused in this step. To the residue were added 220kg of methanol and 10kg of purified water, the mixture was heated to 45 - 64 °C to from a clear solution, and then the solution was cooled to precipitate crystals, centrifuged and dried at 35 - 40 °C under reduced pressure to dryness to obtain 162.7kg of the compound of formula VI, (3S)-3-[4-[(5-bromo-2-chlorophenyl)methyl]phenoxy] tetrahydrofuran, with a yield of 91.2% and a purity of 99.8%.

### Example 7: Preparation of Dapagliflozin

Using 5-bromo-2-chloro-4'-ethoxydiphenylmethane obtained above, dapagliflozin was prepared with reference to the method in the examples of patent application WO03099836A1 by the following steps:

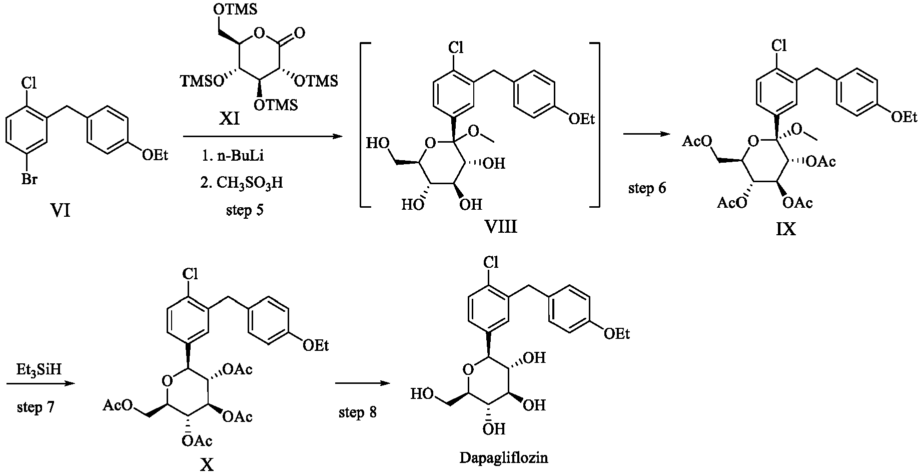

### Synthesis of the compound of formula VIII:

To a reaction flask were added 120g of the compound of formula VI, 330g of tetrahydrofuran and 600g of toluene, the mixture was stirred and cooled to -70°C, and 150 ml of n-butyl lithium was added dropwise while controlling the internal temperature to not exceed -70°C. After dropwise addition was complete, keeping the temperature for 30-60 minutes, and then the mixture was slowly transferred into a reaction flask containing 185g of the compound of formula XI (prepared by the method of step C in the example of WO03099836A 1) and 640g of toluene, controlling the internal temperature to not exceed -70°C. Stirring was continued at this temperature for 3-4 hours, and after the reaction was finished, 55g of methanesulfonic acid and 600g of methanol were added to quench the reaction, and the mixture was stirred overnight. Adding a sodium carbonate aqueous solution to adjust the pH to be 6-7, layers were separated, the organic layer was washed with water and concentrated to dryness to obtain 130.5g of the compound of formula VIII with a yield of 80.6% and a purity is 95.1%.

### Synthesis of the compound of formula IX:

To a reaction flask were added 114.4g of the compound of formula VIII, 1200g of toluene and 139g of triethylamine, the mixture was stirred and cooled to 0 °C, 217.5g of acetic anhydride was added and the temperature was maintained for 30 minutes. The cooling was turned off and the mixture was stirred overnight. After the reaction was finished, a H₃PO₄ solution prepared from 270g of 85% H₃PO₄ and 800g of tap water was added to dilute the solution, the solution was stirred for 30 minutes, layers were separated, the aqueous layer was extracted twice with toluene (130g ×2), the organic layers were combined, washed with 150g of tap water, layers were separated, and the organic layer was concentrated to dryness to obtain 150.6g of the compound of formula IX, with a yield of 95.2% and a purity of 97.5%.

### Synthesis of the compound of formula X:

To a reaction flask were added 140g of the compound of formula IX and 800g of acetonitrile, the mixture was stirred and cooled to 0 °C. 60.8g of triethylsilane and 48g of boron trifluoride diethyl etherate were added and the temperature was maintained for 1 hour. The mixture was warmed to room temperature, added with 36g of triethylsilane and 20g of boron trifluoride diethyl etherate, and stirred overnight. After the reaction was finished, the mixture was extracted with 800g of ethyl acetate and washed with saturated sodium bicarbonate solution and tap water in sequence, filtered, and the organic layer was concentrated to dryness under reduced pressure. To the residue was added 560g of ethanol, the mixture was heated to 65 - 75 °C and stirred to form a clear solution. The heating was turned off and stirring was continued overnight, the mixture was filtered and the filter cake was dried to obtain 109.8g of product, with a yield of 82.5% and a purity of 99.4%.

### Synthesis of Dapagliflozin:

To a reaction flask were added 80g of compound of formula X, 200g of tetrahydrofuran, 400g of methanol, and 150g of tap water, the mixture was stirred, 7.2g of lithium hydroxide monohydrate was added, and stirring was continued at room temperature for 8 hours. After the reaction was finished, the mixture was concentrated under reduced pressure to remove the organic solvent, the residue was dissolved in 700g of ethyl acetate, washed with 200g of water, layers were separated, and the organic layer was concentrated to dryness under reduced pressure to obtain 54.2g of dapagliflozin, with a yield of 95.6% and a purity o 99.8%.

## Claims

1. A process for preparing the compound 5-bromo-2-chlorobenzoic acid of formula I, comprising: 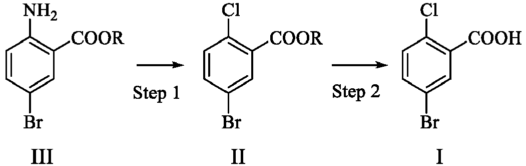
step (1): diazotizing and chlorinating the compound of formula III to prepare the compound of formula II, and
step (2): hydrolyzing the compound of formula II to obtain the compound of formula I,
wherein:
R is selected from: C₁₋₆alkyl, C₂₋₆alkenyl, C₂₋₆alkynyl, C₃₋₈cycloalkyl, C₃₋₈cycloalkyl-C₁₋₆alkyl, C₆₋₁₀aryl, C₆₋₁₀aryl-C₁₋₆alkyl, 5-10 membered heteroaryl, 3-12 membered heterocycloalkyl, 5-10 membered heteroaryl-C₁₋₆alkyl, 3-12 membered heterocycloalkyl-C₁₋₆alkyl, C₁₋₆alkoxycarbonyl, C₁₋₆alkoxycarbonyl-C₁₋₆alkyl, C₆₋₁₀aryl-C₁₋₆alkoxycarbonyl, C₆₋₁₀aryl-C₁₋₆alkoxycarbonyl-C₁₋₆alkyl, each of which is optionally substituted with one or more groups independently selected from halogen, amino, -NH(C₁₋₆alkyl), -N(C₁₋₆alkyl)₂, hydroxy, C₁₋₆alkyl, C₁₋₆alkoxy, halo-C₁₋₆alkyl, halo-C₁₋₆alkoxy, C₃₋₈cycloalkyl, C₆₋₁₀aryl, 5-10 membered heteroaryl, or 3-12 membered heterocycloalkyl;
alternatively, when R is H, step (1) as described above is carried out to directly obtain the compound of formula I.

2. The process according to claim 1, wherein R is selected from: C₁₋₆alkyl, C₂₋₆alkenyl, C₃₋₈cycloalkyl, C₃₋₈cycloalkyl-C₁₋₆alkyl, C₆₋₁₀aryl, or C₆₋₁₀aryl-C₁₋₆alkyl, each of which is optionally substituted with one or more groups independently selected from halogen, amino, -NH(C₁₋₆alkyl), -N(C₁₋₆alkyl)₂, hydroxy, C₁₋₆alkyl, C₁₋₆alkoxy, halo-C₁₋₆alkyl, or halo-C₁₋₆alkoxy.

3. The process according to claim 1, wherein R is selected from: methyl, ethyl, propyl, isopropyl, allyl, cyclopropyl, n-butyl, isobutyl, tert-butyl, cyclobutyl, n-pentyl, n-hexyl, cyclohexyl, phenyl or benzyl.

4. The process according to any one of claims 1 to 3, wherein in step (1) the compound of formula III is first diazotized and then chlorinated under the action of a copper catalyst to prepare the compound of formula II.

5. The process according to any one of claims 1 to 3, wherein in step (1) the compound of formula III is subjected to diazotization and chlorination under the action of a copper catalyst to prepare the compound of formula II in a one-pot method.

6. The process according to claim 4 or 5, wherein the copper catalyst is selected from metallic copper, cuprous chloride, cupric chloride, cuprous bromide or cuprous iodide.

7. The process according to any one of claims 1 to 6, wherein in step (1) the diazotization reagent used is selected from nitrous acid or its salts or C₁₋₆alkyl esters, such as nitrous acid, sodium nitrite, potassium nitrite, methyl nitrite, ethyl nitrite, isoamyl nitrite or tert-butyl nitrite.

8. The process according to any one of claims 1 to 7, wherein in step (2) the compound of formula II is hydrolysed in an aqueous alkali metal hydroxide solution to obtain the compound of formula I.

9. A process for preparing the compound of formula III, comprising: 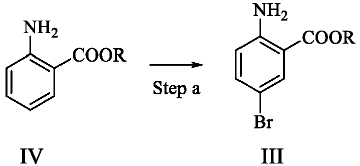
step (a): reacting the compound of formula IV with a brominating reagent to obtain the compound of formula III,
wherein R is as defined in any one of claims 1 to 3.

10. The process according to claim 9, wherein said brominating reagent is selected from bromine, hydrobromic acid, lithium bromide, sodium bromide, potassium bromide, sodium bromate, dibromohydantoin, N-bromoacetamide, N-bromosuccinimide, phenyltrimethylammonium tribromide, (bromomethyl) triphenylphosphonium bromide, or a mixture of two or more thereof.

11. A process for preparing the compound 5-bromo-2-chlorobenzoic acid of formula I, comprising the steps (a), (1) and (2): 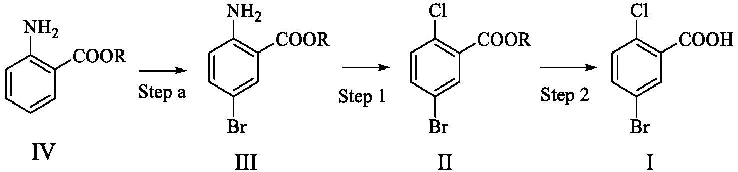 wherein R is as defined in any one of claims 1 to 3, step (a) is as defined in claim 9 or 10, and steps (1) and (2) are as defined in any one of claims 1 to 8.

12. A process for preparing the compound of formula VI, comprising the steps of: 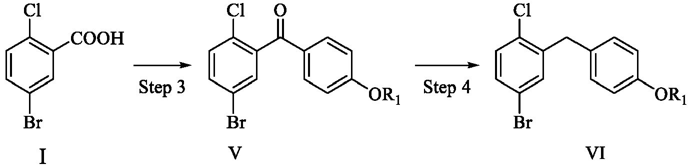 step (3): reacting the compound of formula I with the compound of formula VII through Friedel-crafts acylation reaction to prepare the compound of formula V, 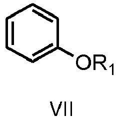
wherein R₁ is selected from: H, C₁₋₆alkyl, such as methyl or ethyl, 3-tetrahydrofuranyl, 3-S-tetrahydrofuranyl or 3-R-tetrahydrofuranyl; and
step (4): reducing the compound of formula V to obtain the compound of formula VI.

13. The process for preparing the compound of formula VI according to claim 12, comprising steps (1), (2), (3) and (4): 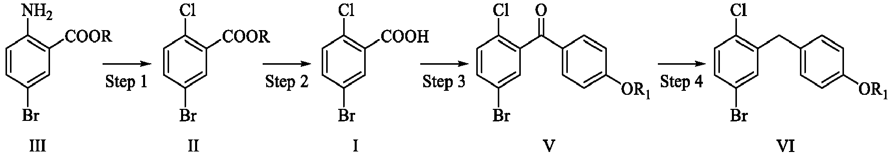
(1) diazotizing and chlorinating the compound of formula III to prepare the compound of formula II,
(2) hydrolyzing the compound of formula II to obtain the compound of formula I,
(3) reacting the compound of formula I with the compound of formula VII through Friedel-crafts acylation reaction to prepare the compound of formula V, and
(4) reducing the compound of formula V to obtain the compound of formula VI,
wherein R is as defined in any one of claims 1 to 3, R₁ is selected from H, C₁₋₆alkyl, such as methyl or ethyl, 3-tetrahydrofuranyl, 3-S-tetrahydrofuranyl or 3-R-tetrahydrofuranyl.

14. The process according to claim 13, wherein in step (1), the compound of formula III is first diazotized and then chlorinated under the action of a copper catalyst to prepare the compound of formula II, or the compound of formula III is subjected to diazotization and chlorination under the action of a copper catalyst to prepare the compound of formula II in a one-pot method.

15. The process according to any one of claims 12 to 14, wherein in step (3) the compound of formula I is converted into an acyl chloride under the action of an acylating reagent in a weakly polar solvent, and then reacted with the compound of formula VII in a weakly polar solvent under the action of a Lewis acid to obtain the compound of formula V.

16. The process according to any one of claims 12 to 15, wherein in step (4) the compound of formula V is reduced in the presence of a reducing agent and an adjuvant to give the compound of formula VI.

17. A process for preparing the compound of formula VI, comprising steps (a), (1), (2), (3) and (4): 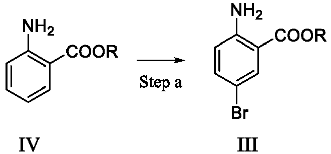 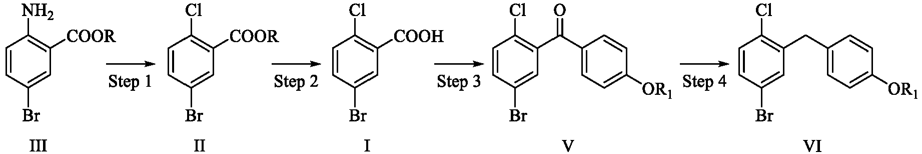 wherein R is as defined in any one of claims 1 to 3, R₁ is selected from H, C₁₋₆alkyl, such as methyl or ethyl, 3-tetrahydrofuranyl, 3-S-tetrahydrofuranyl or 3-R-tetrahydrofuranyl, wherein steps (a), (1), (2), (3) and (4) are as defined in the preceding claims.

18. Use of the compound of formula III as claimed in claim 1 for the preparation of dapagliflozin and engagliflozin.

19. A process for preparing dapagliflozin, comprising any one or more of steps (a), (1), (2), (3) and (4) according to any one of claims 1 to 17.
